# EUROPEAN PATENT SPECIFICATION

(11) **EP 1 372 761 B1**
(45) Date of publication and mention of the grant of the patent: **30.11.2005**
(21) Application number: 02728017.1
(22) Date of filing: 02.04.2002
(51) Int. Cl.: A61M 3/00, A61M 31/00

(54) **DISPOSABLE VAGINAL CANNULA FOR THE SIMULTANEOUS ADMINISTRATION OF DRUGS IN DIFFERENT FORMS**
EINWEG-VAGINALKANÜLE FÜR DIE GLEICHZEITIGE VERABREICHUNG VON ARZNEIMITTELN IN VERSCHIEDENER FORM
CANULE VAGINALE JETABLE POUR L'ADMINISTRATION SIMULTANEE DE MEDICAMENTS DE DIFFERENTES FORMES

(30) Priority: 05.04.2001 IT FO20010010
(43) Date of publication of application: 02.01.2004
(73) Proprietor: Tosato, Luciano, 47900 Rimini (IT)
(72) Inventor: Tosato, Luciano, 47900 Rimini (IT)
(74) Representative: Jappy, John William Graham
(86) International application number: PCT/IT2002/000204
(87) International publication number: WO 2002/081008

(56) References cited:
- AU-B- 545 171
- US-A- 4 341 211
- US-A- 4 439 184

## Description

This invention relates to a device, in particular a disposable plastic cannula, which enables two drugs presented in different physical forms, in particular a gel and a tablet and/or powder, to be delivered into the vagina simultaneously.

The cannula has a particular configuration which enables it to keep the two products constantly separate at the time of packaging and during storage, until the time of use, to prevent deterioration of one or both substances, such as the vaginal tablet, which must not be rehydrated before use, because rehydration causes loss of its therapeutic efficacy or deterioration in its active components.

The cannula in accordance with the invention is particularly practical to use, because the two products are ejected directly into the vagina almost simultaneously with a simple manoeuvre.

Various types of cannula already exist which enable a drug, a douching fluid or the like to be introduced into the vagina.

For example, devices exist which consist of a small plastic reservoir connected to a cannula containing a set of holes in the end through which the product is expelled.

When the cannula is fully screwed onto the body of the container, the end of the cannula perforates a closing wall, thus allowing expulsion of the product, performed by manually compressing the container, which acts as a pump.

***US A 4.341.211*** relates to a device for placing a suppository into a body cavity comprising:
- an applicator cylindrical body with a shaft extending from a first open end to a second open end;
- a compartment or annular chamber surrounding said cylindrical body and filled with a lubricating substance, the wall of said annular chamber having a plurality of openings which are open to the exterior;
- a lubricating plunger sized and shaped to fit within said compartment or annular chamber; and
- a suppository plunger, sized and shaped to fit within said cylindrical body, wherein said suppository plunger, which may push said suppository from said first open end to and out of said second open end when said device is utilized, whereby when said device is placed within said body cavity, said suppository may be pushed through said second open end into said body cavity.

***US A 4.439.184*** describes a syringe for delivering two separated bodies of fluid for use in preparing the urhetra for the insertion of a catheter, comprising:
- a first piston for separating the two bodies of fluid;
- a second piston for pushing the two bodies of fluid out of the syringe; and
- a fluid chamber having a no-pass zone in which the pistons can slide and which has a cross section that each of the pistons substantially fills, preventing the passing of fluid around the pistons when they are in the no-pass zone, an output zone to direct fluid pushed out of the syringe by the pistons, and a bypass zone, for receiving the first piston, which connects the no-pass and output zones and which has a cross section that the first piston is unable to fill, so that when the first piston enters the bypass zone the fluid between the first and second piston can pass around the first piston to the output zone.

The present invention falls into this sector, being designed to solve the problem of administering two products simultaneously, and keeping the said products completely separate until the time of use. The said products may both be semi-solid products; alternatively, as in the case which will be illustrated herein in detail, one product may be in gel form and the other in tablet form.

For this purpose, the invention comprises a cannula fitted at the anterior extremity with a seating designed to house a tablet, a separator able to slide inside the cannula and fitted with means designed to engage the said tablet, a set of holes in a section of the cannula wall upstream of the separator, to allow expulsion of the gel contained in the cannula, and a plunger which expels the gel during the first part of its travel and subseguently engages the separator, causing it to engage with and expel the tablet.

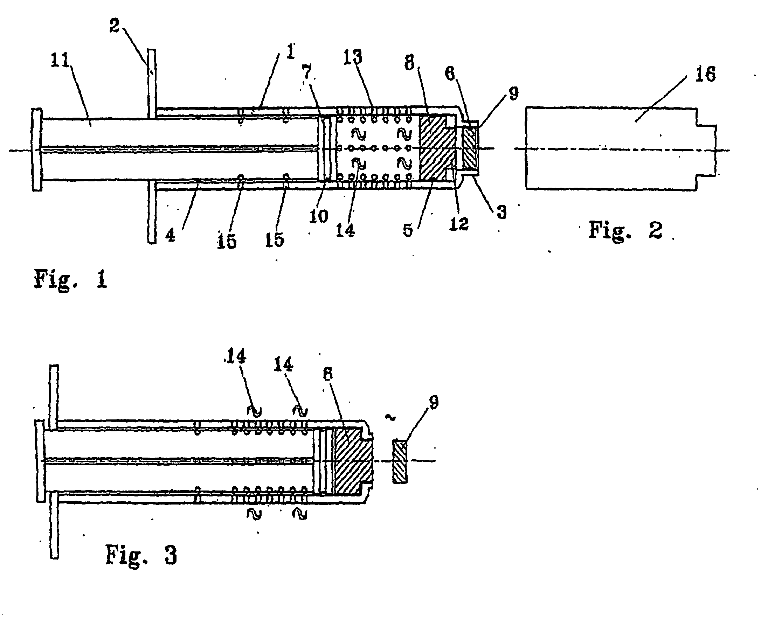

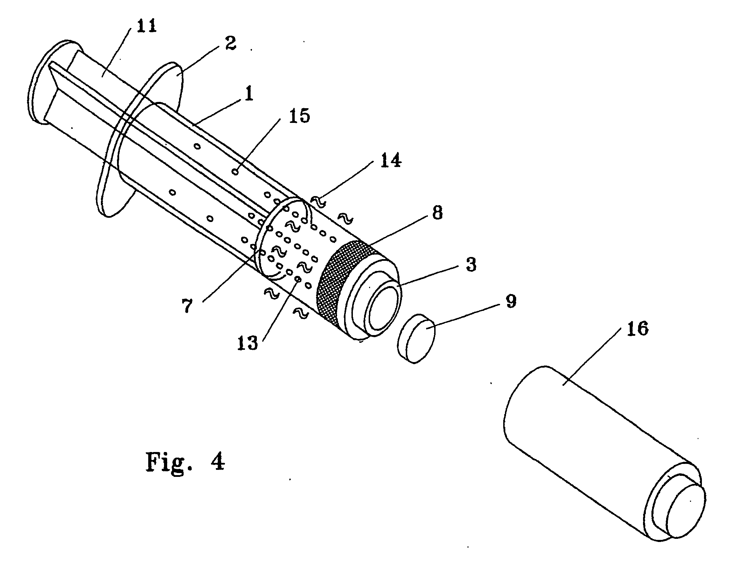

This invention will now be described in detail, by way of example but not of limitation, by reference to the annexed figures in which:
■ figure 1 shows a cross-section of a cannula in accordance with the invention before use, with the packaged product
■ figure 2 shows a cannula sheath which acts as the packaging of the product
■ figure 3 shows a cross-section of a cannula in accordance with the invention after expulsion of the products
■ figure 4 is a perspective view of the cannula in accordance with the invention
■ figure 5 shows a cross-section of a further preferred embodiment of the cannula in accordance with the invention.

As shown in the annexed figures, the cannula in accordance with the invention comprises a barrel 1 which is open at both ends and fitted on one side (the proximal end) with a pair of wings or the like 2, and on the opposite side (the distal end) with a collar of smaller diameter, shown as 3, which surrounds the aperture of the cannula.

On the inner wall of barrel 1 there are three facing ridges shown as nos. 4, 5 and 6 which are designed to engage a plunger 7, a separator/ejector element 8, and a tablet 9, destined to be introduced into the vagina, respectively.

Plunger 7, which slides tightly inside barrel 1, could contain a ring-shaped groove 10 which engages with ridges 4 (or a corresponding raised ring) to retain the plunger and the plunger rod 11 in position before use of the cannula.

Separator 8 consists of a substantially cylindrical body which forms a seal against the inner wall of the cannula and in its anterior part has a cylindrical section 12 of smaller diameter, especially a diameter substantially corresponding to the inner diameter of collar 3.

The length of section 12 is substantially equal to the length of collar 3, so that when separator 8 is in contact with the distal closing wall of the cannula (position illustrated in figure 3), cylindrical body 12 does not project from the cannula.

The body of separator 8 too, will preferably have a ring-shaped groove designed to engage with ridges 5.

In accordance with the invention, the wall of barrel 1 presents a plurality of holes 13 distributed along a length of wall immediately upstream of separator 8, in correspondence with the distal end. These holes allow the expulsion of a semi-solid product, for example a drug or a pharmaceutically acceptable carrier in the form of a gel 14, contained in the cannula.

A second section of cannula wall, upstream of the preceding one, presents a set of holes 15 through which air is expelled from the cannula during the stage of filling the cannula with the drug in gel form, so that plunger 7 can slide until it comes into contact with the gel without any air being trapped.

The cannula is completed by a sheath 16 which acts as the packaging for the product and at the time prevents the gel from leaking out of expulsion holes 13 of the cannula during the filling stage.

The dimensions of the cannula can vary, depending on the material to be delivered, although for most applications its length could be approx. 12 centimetres and its diameter approx. 12-14 mm.

In order to prepare the cannula containing the product, the separator is first inserted into the barrel until it engages with the corresponding ridge 5, which retains it at a distance from the distal end of the cannula, in the position shown in figure 1.

Next, the tablet is introduced by inserting it into collar 3, where it is retained by ridges 6.

Sheath 16 is then fitted over the cannula so as to close the end containing the tablet and cover the entire length containing holes 13.

Next, the required amount of gel is introduced into the cannula, in contact with separator 8, and plunger 7 is inserted and pushed in until it comes into contact with the gel.

The plunger, which is made of plastic suitable for the purpose, slides tightly along the inner wall of the barrel, as in the case of a syringe.

In order to use the cannula it is sufficient to remove sheath 16, hold the cannula by wings 2, and press on rod 11 of plunger 7, which is forced to advance into the cannula.

The thrust exerted by plunger 7 first forces out gel 14 through holes 13 in the cannula wall.

When the plunger has pushed all of the gel out of the cannula, it will come into contact with separator/ejector system 8.

The subsequent advance of the plunger will force the separator out of its seating, pushing it forward until cylindrical body 12 penetrates into collar 3, removing and expelling vaginal tablet 9, which is thus deposited in the vagina.

At this point, separator 8 engages with anterior wall of the cannula, preventing it from advancing further.

The dimensions of ridges 4, 5 and 6, and especially those of ridge 5, which retains separator 8 in position, will be chosen according to the characteristics of the gel, in such a way that the force required to move the separator is greater than that needed to expel the gel.

As will clearly appear from the description supplied, the cannula in accordance with the invention is particularly convenient and practical, since it allows the delivery of two products with different physical characteristics, such as a product in gel form and a solid product in tablet form.

The cannula could also be used to deliver different products for different clinical applications, including, by way of example but not of limitation, the following:
1 - Acidifying gel and vaginal tablet based on *Lactobacillus acidophilus* to rebalance the vaginal ecosystem.
2 - Acidifying gel and metronidazole tablet to treat vaginitis caused by *Gardnerella vaginalis* and *Trichomonas* and simultaneously restore the normal vaginal ecosystem.
3 - Gel with a slightly acid pH and antifungal vaginal tablets to treat fungal vaginitis and provide a soothing effect on the damaged mucosa.
4 - Acidifying gel and antibiotic tablets such as meclocycline to treat bacterial vaginitis and rebalance the vaginal ecosystem.
5 - Oestradiol hormone gel and *Lactobacillus acidophilus* vaginal tablet for topical treatment of the menopause and dystrophic forms, and restoration of the normal vaginal ecosystem.

## Claims

1. Disposable vaginal cannula designed to deliver drugs with different compositions simultaneously, comprising means (1, 3) designed to receive two or more different products to be administered and means (7) designed to expel the said products simultaneously or successively,
- said means (1, 3) designed to receive two or more different products consist of a barrel (1) defining a single cylindrical chamber designed to contain a dose of a first product (14), said chamber having a plurality of holes (13) in the wall of the said barrel (1), in correspondence with the distal section of the cannula, to allow the exit of the said first product (14), and an opening in the anterior wall of the cannula provided with means (3) designed to receive and retain a second medical product (9);
- means (7) designed to expel the said products consist of a plunger (7) designed to press on the said product to control its expulsion;
- a sealed separator (8) being provided, inserted into said chamber in the cannula downstreasn of the said first product (14), said separator (8) sliding inside the cannula and having means (12) designed to engage the second product to control its expulsion.

2. Cannula as claimed in claim 1, **characterised in that** it is provided with a covering sheath (16) designed to be fitted over the distal end of the cannula, which said sheath covers at least the length of cannula containing the said openings (3, 13) for the exit of the product.

3. Vaginal cannula as claimed in each of the preceding claims, **characterised in that** the inner wall of the said cannula presents ridges (5) designed to retain the said separator in position.

4. Cannula as claimed in claim 1, **characterised in that** the inner surface of the cannula presents ridges (6) designed to retain the second product in position.

5. Cannula as claimed in claim 1, **characterised in that** the inner surface of the cannula presents means (4) designed to retain plunger (7), which controls the expulsion of the product, in position prior to use.

6. Cannula as claimed in each of the preceding claims, **characterised in that** the wall of the cannula upstream of the section containing the fist product contains a series of holes (15) designed to allow the exit of air when plunger (7) is introduced into the cannula.

## Patentansprüche

1. Einweg-Vaginalkanüle, welche ausgebildet ist, um gleichzeitig Arzneimittel mit unterschiedlichen Zusammensetzungen zu verabreichen, aufweisend Mittel (1,3), welche ausgebildet sind, um zwei oder mehrere zu verabreichende unterschiedliche Produkte aufzunehmen, und ein Mittel (7), welches ausgebildet ist, um die Produkte gleichzeitig oder nacheinander auszutreiben, wobei
- die Mittel (1,3), welche ausgebildet sind, um zwei oder mehrere unterschiedliche Produkte aufzunehmen, aus einem Zylinder (1), welcher eine einzige zylindrische Kammer definiert, welche ausgebildet ist, um eine Dosis eines ersten Produkts (14) zu enthalten, wobei die Kammer eine Vielzahl von Öffnungen (13) in der Wand des Zylinders (1), in Übereinstimmung mit dem distalen Abschnitt der Kanüle, aufweist, um den Austritt des ersten Produkts (14) zu ermöglichen, und
aus einer Öffnung in der vorderen Wand der Kanüle bestehen, welche mit einem Mittel (3) versehen ist, welches ausgebildet ist, um ein zweites medizinisches Produkt (9) aufzunehmen und aufzubewahren;
- das Mittel (7), welches ausgebildet ist, um die Produkte auszutreiben, aus einem Kolben (7) besteht, welcher ausgebildet ist, um auf das Produkt einen Druck auszuüben, um dessen Austrieb zu steuern;
- eine abgedichtete Trennvorrichtung (8) vorgesehen ist, welche in die Kammer in der Kanüle stromabwärts zu dem ersten Produkt (14) eingefügt ist, wobei die Trennvorrichtung (8) in der Kanüle gleitet und ein Mittel (12) aufweist, welches ausgebildet ist, um mit dem zweiten Produkt in Eingriff zu gelangen, um dessen Austrieb zu steuern.

2. Kanüle nach Anspruch 1, **dadurch gekennzeichnet, dass** diese mit einer Abdeckhülle (16) versehen ist, welche ausgebildet ist, um über das distale Ende der Kanüle zu passen, wobei die Hülle zumindest die Länge der Kanüle abdeckt, welche die Öffnungen (3,13) für den Austrieb des Produkts enthält.

3. Kanüle nach einem der vorhergehenden Ansprüche, **dadurch gekennzeichnet, dass** die innere Wand der Kanüle Rippen (5) aufweist, welche ausgebildet sind, um die Trennvorrichtung in der Position zu halten.

4. Kanüle nach Anspruch 1, **dadurch gekennzeichnet, dass** die innere Oberfläche der Kanüle Rippen (6) aufweist, welche ausgebildet sind, um das zweite Produkt in der Position zu halten.

5. Kanüle nach Anspruch 1, **dadurch gekennzeichnet, dass** die innere Oberfläche der Kanüle ein Mittel (4) aufweist, welches ausgebildet ist, um den Kolben (7), welcher den Austrieb des Produkts steuert, vor der Benutzung in der Position zu halten.

6. Kanüle nach einem der vorhergehenden Ansprüche, **dadurch gekennzeichnet, dass** die Wand der Kanüle stromaufwärts zu dem Abschnitt, welcher das erste Produkt enthält, eine Reihe von Öffnungen (15) enthält, welche ausgebildet sind, um des Austritt von Luft zu ermöglichen, wenn der Kolben (7) in die Kanüle eingeführt wird.

## Revendications

1. Canule vaginale jetable, destinée à l'administration simultanée de médicaments de compositions différentes, comprenant des moyens (1, 3) destinés à recevoir deux produits différents ou plus, à administrer, et des moyens (7) destinés à expulser lesdits produits, simultanément ou successivement,
- lesdits moyens (1, 3) destinés à recevoir deux produits différents ou plus, consistent en un corps cylindrique (1) définissant une seule chambre cylindrique destinée à contenir une dose d'un premier produit (14), ladite chambre ayant une série d'orifices (13) dans la paroi dudit corps cylindrique (1), en correspondance avec la section distale de la canule, pour permettre la sortie dudit premier produit (14) et une ouverture de la paroi antérieure de la canule, procurée par le moyen (3) destiné à recevoir et conserver un deuxième produit médical (9) ;
- les moyens (7) destinés à expulser lesdits produits, consistent en un plongeur (7), destiné à presser sur ledit produit pour contrôler son expulsion ;
- un séparateur scellé (8) étant disposé, inséré dans ladite chambre dans la canule, en aval dudit premier produit (14), ledit séparateur (8) coulissant à l'intérieur de la canule et ayant des moyens (12) destinés à engager le deuxième produit pour contrôler son expulsion.

2. Canule selon la revendication 1, **caractérisée en ce qu'**elle est munie d'une gaine de couverture (16), destinée à être ajustée sur l'extrémité distale de la canule, ladite gaine couvrant au moins la longueur de la canule contenant lesdites ouvertures (3, 13) pour la sortie du produit.

3. Canule vaginale selon chacune des revendications précédentes, **caractérisée en ce que** la paroi interne de ladite canule, présente des nervures (5) destinées à maintenir ledit séparateur en position.

4. Canule selon la revendication 1, **caractérisée en ce que** la surface interne de la canule présente des nervures (6) destinées à conserver le deuxième produit en position.

5. Canule selon la revendication 1, **caractérisée en ce que** la surface interne de la canule, présente des moyens (4) destinés à retenir le plongeur (7), qui contrôle l'expulsion du produit, en position avant utilisation.

6. Canule vaginale selon chacune des revendications précédentes, **caractérisée en ce que** la paroi de la canule en amont de la section contenant le premier produit, contient une série d'orifices (15) destinés à permettre la sortie d'air lorsque le plongeur (7) est introduit dans la canule.
